# EUROPEAN PATENT SPECIFICATION

(11) **EP 3 043 833 B1**
(45) Date of publication and mention of the grant of the patent: **30.05.2018**
(21) Application number: 14761764.1
(22) Date of filing: 25.08.2014
(51) Int. Cl.: A61L 27/18, A61L 27/56, A61L 27/58

(54) **TUBULAR POROUS FOAM SCAFFOLDS WITH GRADIENT PORES FOR TISSUE ENGINEERING**
RÖHRENFÖRMIGE PORÖSE SCHAUMSTOFFGERÜSTE MIT GRADIENTENPOREN ZUR GEWEBEZÜCHTUNG
CHARPENTES EN MOUSSE POREUSE TUBULAIRE À PORES DE TAILLE VARIABLE DESTINÉES AU GÉNIE TISSULAIRE

(30) Priority: 10.09.2013 US 201314022775
(43) Date of publication of application: 20.07.2016
(73) Proprietor: Ethicon, Inc, Somerville, NJ 08876 (US)
(72) Inventor: VALMIKINATHAN, Chandra M., Elmwood Park, New Jersey 07407 (US); CRUZ, Pedro, Elizabeth, New Jersey 07202 (US)
(74) Representative: Carpmaels & Ransford LLP
(86) International application number: PCT/US2014/052469
(87) International publication number: WO 2015/038315

(56) References cited:
- CN-A- 102 727 931
- US-A1- 2008 102 438
- HARLEY B A ET AL: "Fabricating tubular scaffolds with a radial pore size gradient by a spinning technique", BIOMATERIALS, ELSEVIER SCIENCE PUBLISHERS BV., BARKING, GB, vol. 27, no. 6, 1 February 2006 (2006-02-01), pages 866-874, XP027950803, ISSN: 0142-9612 [retrieved on 2006-02-01]

## Description

### Field of the Invention

The field of art to which this invention pertains is polymeric nerve tubes for the regeneration of nerves, more particularly porous, bioabsorbable polymeric nerve tubes providing tissue engineering substrates.

### Background of the Invention

It is known in the medical arts that severed or damaged peripheral nerves often have the ability to regenerate. This inherent regenerative response is dependent upon several factors. If a peripheral nerve is severed fairly cleanly, the nerve ends may reconnect spontaneously via a tissue response wherein axons of neurons bridge the gap to connect the severed ends. This may be facilitated by suturing the ends of the severed nerve together using micro needles and fine sutures. However, it is known that tensioning the nerve ends in order to coapt the nerve stumps for suturing will result in a less than optimal surgical repair in which nerve repair and regeneration will be compromised. In many instances involving trauma, there will be substantial damage to a significant section of a nerve requiring removal of the damaged section. Since the nerve ends should not be tensioned to effect a repair, several surgical procedures have been developed to address these types of trauma situations. Also, in the case of trauma, a significant portion of the nerve may get crushed or cut, precluding the surgeon from connecting or suturing the patient's own nerve ends together. One repair method or procedure for this indication utilizes autographs to essentially fill in the gap between the nerve ends and take the place of the damaged nerve section or sections. The opposed ends of the harvested autograft are then sutured or otherwise attached to the nerve stumps of the damaged nerve to effect a coaptation. A commonly harvested nerve for such reconstructive procedures is the sural nerve. Alternatively, it is known to use allografts and xenografts in the reconstructive repair procedures. However, it is known that there may be problems or complications associated with the use of such grafts. Harvesting the autografts requires an additional incision that may cause pain or provide an entry route for pathogens resulting in localized or systemic infections. In addition, the site of the autograft harvesting may be associated with neuroma and loss of functions at the donor site. The autologous nerve graft may be mismatched in size with the nerve that requires reconstruction, complicating the nerve coaptation procedure and possible affecting the neuroregeneration outcome. Allografts and xenografts may be similarly mismatched in size. It is also known that allografts may be sources of infection if not properly harvested and processed, and may result in tissue rejections. Xenografts must be carefully processed to minimize the immune response to the animal tissue. In addition, it is believed that neural function with a graft does not completely return and is at least somewhat compromised. More importantly, in the case of nerve injuries, the motor neurons are rarely repaired, leading to significantly reduced quality of life.

In order to address some of the problems that may be associated with grafts, nerve tube devices have been developed. The nerve tubes are basically hollow tubes having circular cross-sections. The nerve tubes are made from biocompatible materials including both bioabsorbable materials such as collagen and nonabsorbable materials such as silicone. Nerve tubes may be used in nerve repair procedures where coaptation of the nerve ends is not possible without tension because of the amount of missing nerve in the gap between the ends. This is often the result of trauma wherein a section of the nerve is so severely damaged that it necessarily must be removed. In a typical nerve tube repair procedure, the surgeon will prepare each opposed nerve end surrounding the gap in the nerve in a conventional manner, for example, the damaged nerve is cleaned, debrided and prepared for surgery. Each nerve end is then inserted into an open end of the nerve tube and sutured to the nerve tube using surgical sutures. Over time, the lumen of the nerve tube fills with fluid and subsequently a fibrin matrix is formed between the nerve ends or stumps. Over the course of several months, neurons regenerate, and the regenerated neurons extend from one nerve stump into the fibrin matrix and over to the other stump, connecting the ends of the nerve and forming a new nerve segment to replace the excised nerve segment. It is known that conventional nerve tubes have their limitations as well, including being limited to regenerating nerves when gaps are no larger than 30 mm in length. In addition, it is believed that complete nerve function may not be restored, while rogue neurons may innervate tissue adjacent to the nerve tube implant. For a nerve tube implant procedure to achieve maximum nerve regeneration and recovery, it is known that the nerve tube must have several critical properties. First of all the nerve tube must provide an environment wherein bodily fluids may enter into the lumen of the nerve tube to permit the transport of necessary nutrients and the elimination of cellular waste products. The nerve tube at the same time must protect the nerve stumps from attack by immune cells such as macrophages and T-cells, and must also protect the nerve stumps from fibroblasts, which can lead to significant scar tissue formation, leading to reduced regenerative capacity. It is also desirable for the best long term prognosis that the nerve tubes be made from bioabsorbable polymers. Such bioabsorbable nerve tubes retain their functional qualities during the neuroregeneration process, and then degrade and are eliminated from the body in order to return the traumatized body site to a normal condition. In addition, it is desirable for bioabsorbable nerve tubes to have a porous structure to allow nerve cells, blood vessels and glial cells to grow into the structure to provide a more optimal nerve regeneration. The porous structure will also allow the inflow and outflow of bodily fluids containing ions, salts, proteins, nutrients, and cellular waste products while preventing the migration of inflammatory cells into the nerve tube structure.

Although the nerve tubes that are known in the art are satisfactory for their intended use, it is known that there are problems or deficiencies associated with these nerve tubes and their use in nerve repair procedures. One deficiency relates to the limitation in the lengths of the nerve gaps that can be repaired, conventionally thought to be about 30 mm. The deficiencies also include insufficient nutrient influx and/or metabolic waste removal, leading to reduced repair of nerve gaps, which eventually causes distal neural tissue death; and, inflammation within the tube leading to misguided neurons, which may not reach the ends of the tube and thereby not connect the nerve stumps to effect an adequate repair of the nerve. In addition, porous nerve tubes of the prior art are known to have several deficiencies. In order to have a gradated and controlled pore size architecture, it has been necessary to utilize multiple casting steps using more than one polymer. In addition, the use of salts in a polymer matrix during casting is known, but the salts must be leached away after casting to form the porous structure and the use of such salts is unacceptable with bioabsorbable polymers. Also, generating gradients radially, with a controlled wall-like architecture, on the outside, is challenging with contemporary technologies.

US 2008/1024238 A1 concerns a process for fabricating molded structures having a radially organized pore structure. Harley B A et al: "Fabricating tubular scaffold with radial pore size gradient by spinning technique", Biomaterials, Elsevier, vol. 27, no. 6, 1 Feb 2006, pages 866-874 concerns a fabrication process to produce collagen-based, porous tubular scaffolds. CN 102 727 931 A concerns the construction and preparation of a three-dimensional bionic electropolarized gradient pore nerve conduit.

Accordingly, there is a need in this art for novel nerve tubes made from bioabsorbable porous foam polymeric structures that promote the regeneration of severed or damaged nerves, and novel processes for forming such nerve tubes.

### Summary of the Invention

The present invention provides a porous, bioabsorbable nerve tube structure according to claim 1. In one embodiment, the tube structure has a bioabsorbable polymeric tubular foam member. The tubular member has a lumen, an inner surface surrounding the lumen, and an outer surface defining a wall. The wall has a thickness. The tubular member has opposed ends, and an opening in each end in communication with the lumen. The foam member has a gradient of pores from the interior surface to the exterior surface across the wall thickness of the tubular foam member such that tissue cells may infiltrate from the inner surface into the wall, and the outer surface is impermeable by inflammatory cells. The wall is permeable by fluids passing through the wall.

Another aspect of the present invention is a method of making a porous, bioabsorbable nerve tube structure according to claim 2. In one embodiment, the method includes the following steps. A bioabsorbable polymer solution is provided. The polymer solution is spin cast to form a tubular structure. The tubular structure is lyophilized to form a porous, bioabsorbable polymeric tubular foam structure having a foam member with an inner surface surrounding a lumen, an outer surface, and a wall thickness between the inner and outer surfaces. The foam structure has a gradient in pore sizes between the inner and outer surfaces. The inner surfaces allow cellular infiltration into the wall, while the outer surfaces do not allow cellular growth into the wall. The wall is permeable by fluids passing through the wall.

These and other aspects and advantages of the present invention will become more apparent from the following description and accompanying drawings.

### Brief Description of the Drawings

FIG. 1 is a scanning electron microphotograph illustrating a cross-section of nerve tubes of the present invention, with gradient pore architecture.
FIG. 2 is an optical microphotograph illustrating several nerve tubes prepared using several polymers and processes of the present invention.
FIG. 3 is a microphotograph illustrating cellular ingrowth into the interior wall of a nerve tube structure of Example 1; no cells are seen to penetrate through the outer surface of the nerve tube.
FIG. 4 is a microphotograph showing pore interconnectivity and the varying wettability of the inner and outer surfaces of a nerve tube of the present invention due to the pore dimensions and configuration.

### Detailed Description of the Invention

The novel nerve tubes of the present invention are made from conventional bioabsorbable polymers and copolymers that have sufficiently effective solubility in common solvents that are used in lyophilization processes. The term bioabsorbable is meant to be a generic term, which may also include absorbable, resorbable, bioresorbable, degradable, and biodegradable. The bioabsorbable polymers include but are not limited to polycaprolactone (PCL), polydioxanone (PDO), polylactic acid (PLA), polyglycolic acid (PGA), polyurethanes, and the like and copolymers thereof. Also, several natural polymers such as collagen, gelatin, chitosan, hyaluronic acid, fibrin, elastin, etc., can also be fabricated into the novel nerve tubes of the present invention using the method of the present invention. The term polymer as used herein is defined to include homopolymers, copolymers, and mixtures thereof (natural and synthetic).

Several therapeutic agents such as growth factors, small molecules, primary autologous cell extracts, stem cell produced factors and extracts, blood-based components such as platelet extract, peptides and proteins can also be added to the tubes during the processing steps. The proteins can be added either during the tube fabrication process (spinning) or after lyophilization, such that the above components can be absorbed into the foams and can be re-lyophilized to obtain dry tubes for implantation. If desired, these components can also be added (soaked) just prior to implantation as well.

The solvents that can be used to form the polymer solutions used in the novel manufacturing processes of the present invention include conventional organic solvents that are known for use in conventional lyophilizing processes. The solvents include 1,4-dioxane, dichloro methane, chloroform, dimethyl sulfoxide and the like and mixtures thereof.

The polymers solutions useful in the methods of the present invention will have a sufficient concentration of the bioabsorbable polymer to be effectively flowable into the casting tubes as well as provide sufficient viscosity to gel along the interior walls of the tubes. The concentration will depend upon several factors including required viscosity, dimensions of tube required, spinning speed and conditions, and temperature. For example, and not meant to be limiting, the concentration of the polymer component in the solution will typically be about 1 wt.% to about 25 wt.%, more typically about 8 wt.% to about 20 wt.%, and preferably 12 wt.% to about 16 wt.%. The concentrations of the polymer solutions used to manufacture the nerve tubes of the present invention will be sufficient to provide for effective flow into the tube and high enough viscosity to prevent flow outside of the tube

The nerve tubes of the present invention may contain conventional medically useful substances including, but not limited to, antimicrobial agents, antibiotics, growth factors, and therapeutic agents. The antimicrobial agents may include, for example, peptide-based antibacterials, triclosan, and other small molecules. The antibiotics may include, for example, penicillin and other biologic antibiotics, etc. In addition, some natural polymers such as chitosan that are known to be antibacterial can also be blended into the nerve tube matrix.

The novel nerve tubes of the present invention are made in accordance with the following novel process. Initially, a bioabsorbable polymer solution is prepared and made by weighing out a quantity of a suitable bioabsorbable polymer. The bioabsorbable polymer is added to a sufficient amount of a suitable solvent and poured into a conventional, heated mixing vessel to provide a polymer solution having the desired concentration. The solution is stirred and heated in a conventional mixing vessel at a sufficiently effective temperature for a sufficiently effective period of time to completely dissolve the polymer. The solution is then ready for centrifugal casting in order to form the cast nerve tubes. Using a conventional centrifugal casting apparatus as described below, a suitable casting tube is selected having the desired inner diameter. The polymer solution is then aspirated into the lumen of the casting tube in a conventional manner, for example, when using a casting tube that is a conventional glass pipette, the polymeric casting solution is aspirated into a 3, 5 or 10 ml glass pipette using a pipette aid, with a cotton tipped back end intact to prevent leak of the solution after it is set for spinning, while allowing for air flow into the tube, in order to dry the solution. The casting tube containing the polymer solution is then mounted to the drive mechanism of the casting apparatus and rotated at a sufficiently effective angular speed for a sufficiently effective period of time to evaporate a significant amount of the solvent and form a gel-like coating on the glass walls of the pipette. The angular speed and the time selected will depend on several factors, including but not limited to the viscosity of the solution, dimensions of the tube, solvent applied, temperature, and the polymer used.

The angular speed is 800 rpm to 3,000 rpm, more typically about 1500 rpm to about 2500 rpm, and preferably about 2,000 rpm to about 2,500 rpm. Additionally, the casting time is 12 hours to 48 hours, more typically about 24 hours to about 48 hours, and preferably be about 24 hours to about 36 hours.

After spin casting has been completed, the system, including the casting tube and the cast polymer, is removed from the casting apparatus and moved to a conventional lyophilization apparatus. The lyophilization process is conducted in the following manner. The tube is dismounted from the drive mechanism and preferably placed on insulating blocks to prevent direct contact of the tube with the plates of the lyophilizer, which could possibly lead to direct heat transfer. The plates may be precooled to induce different pore structure formation as desired. By changing the precool temperature, the pore architecture and thickness of the zones (high and low porosity) may potentially be controlled.

The process preferably requires the precooling of the plates to the desired sufficiently effective temperature (for example, anywhere from about +50° C to about -50° C). The interim stage cast polymer nerve tubes contained within the casting tubes, are placed inside the lyophilization chamber without directly touching the metal floors or elevated on Teflon blocks or other insulating blocks. The interim stage nerve tubes contained in the casting tubes are lyophilized with an appropriate cycle and conditions for a sufficiently effective time and sufficiently effective temperature(s) and pressure(s) to completely remove the solvent from the solution, leaving behind dry tubes, for example the lyophilization time may range from about 24 hours to about 48 hours. After completion of the lyophilization cycle, the casting tubes containing the lyophilized and dried nerve tubes having a porous structure are removed from the chamber, and the lyophilized tubes are extracted from the casting tubes.

The novel porous nerve tubes of the present invention may be made in a variety of sizes and lengths to accommodate different nerve sizes and various gaps between nerve stumps. Typically, and in general, the nerve tube selected is sufficiently sized to effectively bridge peripheral nerve gaps of all sizes (e.g., from 5 mm to 50 mm or larger) and nerves having various diameters (e.g., from about 1 mm to about 10 mm diameter). For example, nerve tubes of several dimensions from 2 mm inner diameter to 5 mm inner diameter can used for several applications including facial nerves damaged during plastic surgery or large nerve injuries that occur due to trauma or tumor resection. Although the nerve tubes of the present invention may have a lumen with a wide range of inner and outer diameter sizes, the nerve tubes will typically have a diameter of the inner lumen of typically about 1 mm to about 7 mm, more typically about 2 mm to about 5 mm, and preferably about 2.5 mm to about 5 mm. The outer diameter of the nerve tubes will typically range from about 2.5 mm to about 9 mm, more typically about 3 mm to about 7 mm and preferably about 3 mm to about 6 mm. The thickness of the walls of the nerve tubes of the present invention will be sufficient to effectively provide the desired mechanical properties, such as the ability to resist tension, allow for suture hold, provide the desired rigidity, and prevent migration of inflammatory cells into the regeneration space of the scaffold. For example, the wall thickness may range from about 1 mm to about 4 mm, more typically about 1mm to about 3 mm, and preferably about1 mm to about 2.5 mm. Alternatively, and for example, the wall thickness may be between about 0.5 mm and about 3 mm, more typically about 1 mm to about 2.5 mm, and preferably about 1mm to about 2 mm. The lengths of the nerve tubes of the present invention will be sufficient to provide enough length to effectively bridge a nerve gap. It is contemplated that the nerve tubes may come in a standard length that may be trimmed and adjusted by the surgeon during a surgical procedure to accommodate a particular nerve gap, or the nerve tubes may be made by the manufacturer in a variety of lengths. For examples, the nerve tubes of the present invention may have a length ranging from about 3 mm or shorter to about 5 cm or longer.

The nerve tubes of the present invention will have a gradient in pore size from larger to smaller across the wall thickness from the interior wall surrounding the lumen to the outer wall of the nerve tube. The pore size gradient will be sufficiently effective to prevent inflammatory cell migration, meanwhile allow for nutrient exchange across the walls. In addition, the larger inner pores will allow for new blood vessel formation and neural cell ingrowth. The pore sizes moving from the inner surface of the tube wall to the outer surface will range from about 100 microns to about 1 micron, more typically about 50 microns to about 1 micron, and preferably about 50 microns to 1 micron.

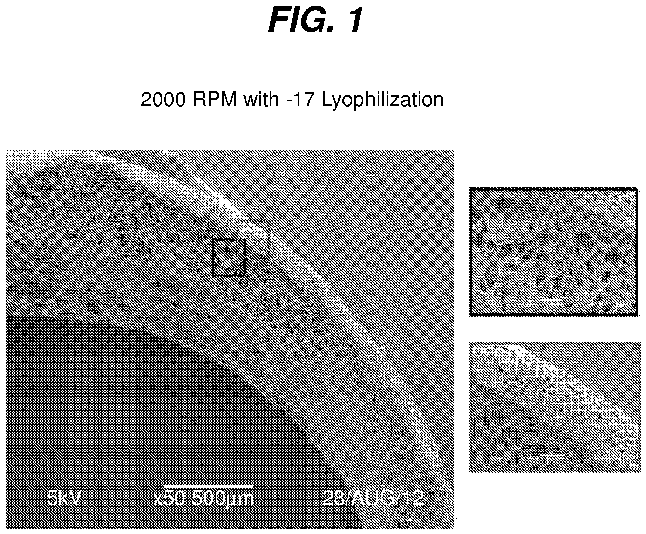

It can be observed from FIG. 1, that there exists a distinct interface between the high and the low porosity regions. This interface serves, among other things, as a barrier to migration of inflammatory cells from the outside, meanwhile the interface does not interfere with nutrient penetration and waste removal into and from the scaffolds respectively. However, as the nerve regenerates, the interface becomes more and more porous, owing to the degradability of the polymer, therefore allowing for complete neural cell infiltration and remodeling of the injured nerve.

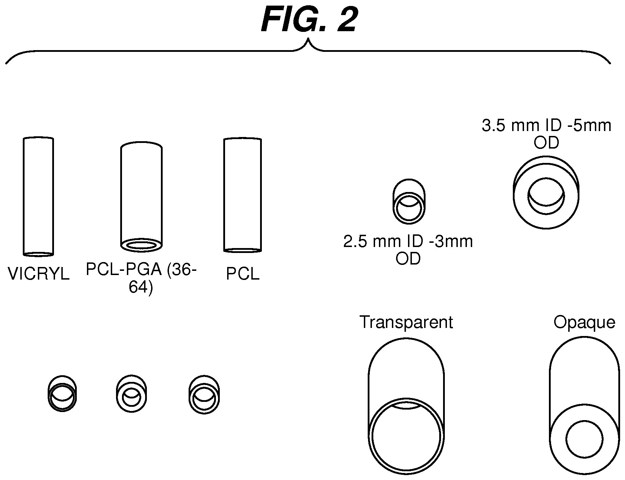

Referring to FIG. 2, images of various nerve tubes of the present invention are seen. The images on the left show tubes with the same dimensions made using different polymers (Vicryl ®, PCL-PGA 36-64 and Polycaprolactone (PCL). The image on the top right shows tubes made from PCL-PGA with different dimensions suitable for implantation. The image on right bottom shows transparent tubes, composed of PCL-PGA, prepared without a lyophilization cycle (transparent) and using lyophilization (opaque). Here, can be seen the effect of generating pores using the lyophilization process, leading to "foam-like" architecture with a thicker wall structure.

If desired, the nerve tubes of the present invention may have a variety of compositions or materials inserted into the lumens in order to promote nerve growth and regeneration. The compositions or materials may include bioloigics, collagen gels, fibrin, hyaluronic acid, gelatin, other protein/ peptide-based gels as well as gels composed of growth factors and matrix proteins such as fibronectin, laminin, collagen or other such proteins. The gels can also be loaded with antimicrobial agents or small molecules to induce specific response such as enhanced wound healing, increased neurite outgrowth, etc. The materials or compositions are inserted into the lumens of the nerve tubes of the present invention in a conventional manner such as, for example, the gels can prefilled into the tubes, prior to lyophilization, or after lyophilization, filled and then re-lyophilized, or may be filled in the field prior to implantation. Also, specific patterns such as randomly or aligned pores within the tubes can be fabricated by controlled cooling or lyophilization processes. The interior lumens may if desired have an incorporated structure, including channels, fibers, hydrogels and porous foams.

The centrifugal casting apparatuses useful in the processes of the present invention to manufacture the novel porous nerve tubes of the present invention are conventional, commercially available apparatuses that provide a mounting chuck and a motor and drive to spin the chuck at a present angular speed. An example of such an apparatus is a vertical stirrer used in solvent mixing or homogenization. One apparatus useful in the process of the present invention is the IKA Eurostar digital stirrer, with a maximum torque of 20 Nm and a maximum speed of 6000 RPM. The centrifugal casting apparatuses typically operate in the following manner. The chuck or other engagement device is adjusted to secure and hold the casting tube (e.g., glass pipette), which is tightened and the motor is turned on. The casting tube may be optionally initially slightly tilted, so that entire wall of the tube is filled with the polymer solution and the speed of the motor is taken up gradually to the desired rate (e.g., typically about 2000 RPM to about 2500 RPM).

The lyophilization apparatuses useful in the practice of the present invention are conventional, commercially available lyophilization units that provide for sufficiently effective freeze and vacuum cycles. An example of such a commercially available conventional lyophilization unit is a Virtis lyophilization model typically used for lyophilization of drugs and proteins in the pharmaceutical industries. Typically, a tray style lyophilizer is preferred.

The lyophilization units will be sufficiently effective to completely remove the solvent and keep the tubes intact at required temperatures and pressures. The units will typically have the following temperature and vacuum parameters ranging from about -50°C to about 50°C and a pressure level up to 0.133 Pa (0.001 Torr).

Although it is preferred to use glass pipettes as the casting tubes, the casting tubes may be made from other conventional materials, including polymers such as Teflon, and polyethylene; and, metals and alloys such as stainless steel, copper etc. The material selected for the casting tube depends on several factors, including the solvent used for the polymer of choice.

The following examples are illustrative of the principles and practice of the present invention, although not limited thereto.

### Example 1

### Processing Method for Peripheral Nerve Tubes (PNTs)

This example describes the solution and centrifugal casting steps of the novel process used to manufacture the novel nerve tubes of the present invention.

### Polymer Solution

A solution composed of a caprolactone and glycolide copolymer (PCL/PGA) in 1-4 Dioxane was prepared and added to a conventional vessel having a conventional mixer. The solution contained 12 weight percent of the copolymer. The solution was heated for a period of 2 hours in a water bath maintained at 70 degrees Celsius. During this time, the solution was mixed with the aid of the mixer. After the polymer was dissolved in the solvent, the solution was filtered by salt filter to remove undissolved polymer and debris. After filtering the solution, care was taken to keep the solution from cooling down by maintaining the solution in a heated water bath maintained at 50-60 degrees Celsius.

### Centrifugal Casting

The solution was heated to a temperature between 60-70 degrees Celsius. A pipette was used to centrifugally cast a cylindrical tube composed of the polymer solution. The pipette had a capacity of 5 ml (from 2ml to 10 mL can be used). Glass pipettes were preferred because of the solvents used and since the length can be varied to get appropriate nerve gap sizes. The ID of the tube was about 5 mm and the OD was about 7 mm. 3.5ml of polymer solution was aspirated into the lumen of the pipette. The amount of polymer solution that is aspirated will dictate the thickness of the cylinder wall created. Upon completing this step, the pipette containing the polymer solution was mounted and inserted to the chuck of a conventional drill. The drill was mounted to a conventional overhead spinner with a maximum spinning speed of 2500 RPM in the following manner. The chuck was loosened to make sure the glass pipette fit in the gap. The polymer solution was loaded into the glass pipette and an end of the tube was loaded in the chuck. The chuck was tightened to ensure that the pipette was secure and would not slip. The drill was then turned on and set to a rotating speed of 2000 rpm. Initially, some solution leaked from the pipette ends, but this leakage stopped as the solution began to gel with resultant capping of the open ends of the pipette. The rotation of the pipette also helped to prevent the polymer solution from leaking. In order to create a tube that is approximately the length of the pipette, it is necessary that the solution covers the majority of the length of the pipette. In this example during the first minutes of rotation, the rotation equipment was (optionally) angled downward from the horizontal position to allow the solution to cover the length of the casting tube.

### Lyophilization

The spin cast polymer solutions (i.e., interim nerve tubes) in the casting tubes were then lyophilized using a conventional lyophilizer: SP Scientific Virtis (Brand), Lyostar 3 (model) tray type lyophilizer. The lyophilization protocol is presented in Table 1.

**Table 1**

| Segment | Time (min) | Temperature (degrees Celsius) | Pressure (Pa) |
|---|---|---|---|
| Freezing | 120 | -17 | |
| Drying | 600 | -5 | 13.33 (100 mT) |
| Drying | 300 | 5 | 2.66 (20 mT) |
| Drying | 240 | 20 | 2.66 (20 mT) |

Additional nerve tubes of the present invention were then made in accordance with the following Examples.

### Example 2

A 20% polymer solution prepared in accordance with Example 1 was centrifugally cast in accordance with Example 1 at 2000 rpm. Into a 5ml tube, 3.5ml of the polymer solution aspirated at a constant speed of 2031 rpm. The tube containing the polymer solution was centrifugally cast for a period of 36 hours. The system consisting of the tube and the cast polymer nerve tube was loaded onto the freeze-drying machine. The shelves were initially at room temperature. The shelves were cooled down to a temperature of -35 degrees Celsius and then maintained at this temperature for a period of two hours. After this freezing phase, the chamber pressure was reduced to 13.33 Pa (100 milliTorr (mT)) and the shelf temperature was brought up to -17 degree Celsius and maintained at this for a period of 560 minutes. The pressure within the chamber was further reduced to 2.66 Pa (20 mT) and the temperature was increased to 10 degrees Celsius and maintained for 240 minutes. The chamber was maintained at 2.66 Pa (20 mT) and the shelf temperature was increased to 20 degrees Celsius and held at this temperature for a period of 60 minutes to complete the cycle.

At the end of the cycle, the chamber was purged with nitrogen and atmospheric pressure was restored. The tube system was then removed from the lyophilizer and then ethanol was lightly sprayed into the lumen of the tube to aid in the removal of the lyophilized nerve tube from the casting tube. After the nerve tube was removed, the nerve tube was placed in a laboratory hood to allow the ethanol to evaporate.

### Example 3

A 20% polymer caprolactone and glycolide copolymer (PCL/PGA) solution was prepared in accordance with Example 1 and centrifugally cast at 2056 rpm for a period of 24 hours in accordance with Example 1. Into a 5ml casting tube, 2.5ml of the polymer solution was aspirated at a constant speed of 2050 rpm. The casting tube containing the polymer solution was centrifugally cast for a period of 24 hours. The system consisting of the casting tube and the cast polymer nerve tube was loaded onto the freeze-drying apparatus maintained at -17°C. The cast polymer then was lyophilized. The lyophilization protocol used is detailed in Table 1.

With the cycle completed, the chamber was purged with nitrogen and atmospheric pressure was restored. The tube system was then removed from the lyophilizer and then ethanol was lightly sprayed into the lumen of the tube to aid in the removal of the lyophilized nerve tube from the casting tube. After the nerve tube was removed, the nerve tube was placed in a laboratory hood to allow the ethanol to evaporate. It was observed (see FIG. 1) that the use of pre-cooled shelves altered microstructures of the lyophilized tube and created a finer pore structure.

### Example 4

A (15 wt.%)Vicryl® (polyglactin 910) polymer solution was prepared in accordance with procedure of Example 1 except that the solution was heated to 60°C. The polymer solution was centrifugally cast at 2000 rpm for a period of 24 hours in accordance with Example 1. Into a 5ml casting tube, 3.0 ml of the polymer solution aspirated at a constant speed of 2080 rpm. The casting tube containing the polymer solution was centrifugally cast for a period of 24 hours. The system consisting of the casting tube and the cast polymer nerve tube was loaded onto the freeze - drying apparatus maintained at 25°C. The cast polymer then was lyophilized. The lyophilization protocol is detailed in Table 2.

**Table 2**

| Segment | Time (min) | Temperature (degrees Celsius) | Pressure (Pa) |
|---|---|---|---|
| Freezing | 120 | -50 | |
| Drying | 540 | -17 | 13.33 (100 mT) |
| Drying | 240 | 10 | 2.66 (20 mT) |
| Drying | 120 | 20 | 2.66 (20 mT) |

With the cycle completed, the chamber was purged with nitrogen and atmospheric pressure was restored. The tube system was then removed from the lyophilizer and then ethanol was lightly sprayed into the lumen of the tube to aid in the removal of the lyophilized nerve tube from the casting tube. After the nerve tube was removed, the nerve tube was placed in a laboratory hood to allow the ethanol to evaporate. The lyophilized tube was observed to be stiffer than tubes described in Examples 2 and 3.

### Example 5

A 20 wt.% polycaprolactone polymer solution was prepared in accordance with procedure of Example 1 except that the solution was heated to 60°C. The polymer solution was centrifugally cast at 2000 rpm for a period of 24 hours in accordance with Example 1. Into a 5 ml casting tube, 3.0 ml of the polymer solution was aspirated at a constant speed of 2000 rpm. The tube containing the polymer solution was centrifugally cast for a period of 24 hours. The system consisting of the tube and the cast polymer nerve tube was loaded onto the freeze-drying apparatus maintained at 25°C. The cast polymer then was lyophilized. The lyophilization protocol utilized is detailed in Table 2.

With the cycle completed, the chamber was purged with nitrogen and atmospheric pressure was restored. The tube system was then removed from the lyophilizer and then ethanol was lightly sprayed into the lumen of the tube to aid in the removal of the lyophilized nerve tube from the casting tube. After the nerve tube was removed, the nerve tube was placed in a laboratory hood to allow the ethanol to evaporate. The lyophilized tube was observed to be stiffer than tubes described in Examples 2 and 3. It was observed that the tube did not exhibit elastomeric properties. The tube was observed to be pliable but not stiff, and would not retract after applying force.

### Example 6

A 20 wt.% polycaprolactone/PGA copolymer solution was prepared in accordance with procedure of Example 1 except that the solution was heated to 60°C. The polymer solution was centrifugally cast at 2000 rpm for a period of 24 hours in accordance with Example 1. Into a 1 ml casting tube, 0.55 ml of the polymer solution was aspirated at a constant speed of 2,500 rpm. The casting tube containing the polymer solution was centrifugally cast for a period of 2.0 hours. The system consisting of the casting tube and the cast polymer nerve tube was loaded onto the freeze-drying machine maintained at -17°C. The cast polymer then was lyophilized in accordance with the lyophilization protocol of Example 1. It was observed that a small diameter nerve tube of the present invention having the desired porous structure was obtained. The tube was observed to be adhered to the glass wall and removal was aided by the use of IPA. After the nerve tube was removed, the nerve tube was placed in a laboratory hood to allow the IPA to evaporate.

### Example 7

### Cellular Ingrowth Testing

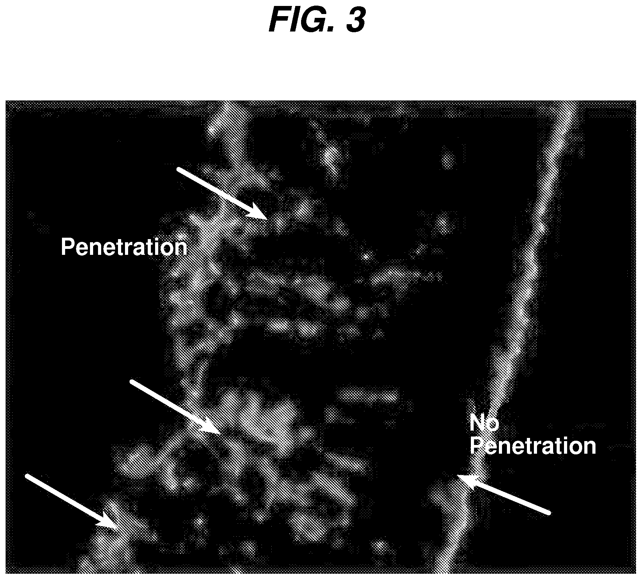

Nerve tubes fabricated from 20% PCL-PGA polymer, fabricated with lyophilization precool temperature of -17°C, manufactured in accordance with Example 1, were tested for cell ingrowth in the following manner. The tubes were cut into 1 cm lengths and were sterilized in 70% ethanol for 30 minutes. The lengths of tubes were then placed in sterile phosphate buffered saline (PBS) for 3 hours with new solution every hour. The tubes were then washed thoroughly in cell culture media. In a new 15 ml centrifuge tube, 2 million cells were placed in 2 ml complete medium and the tubes were immersed for 4 hours. The tubes were then removed and transferred to a six well plate containing complete medium and were allowed to grow for 48 hours. After 48 hours, the tubes were fixed in 4% paraformaldehyde and washed in PBS after 2 hours. The tubes were sectioned after embedding in optimal cutting temperature compound (OCT) and 10 micron sections were stained with actin (phalloidin Texas red) and nuclear stain 4',6-diamidino-2-phenylindole (DAPI) and imaged. From the data, shown as FIG. 3, it was seen that cell penetration from the outer wall inward was absent. Meanwhile, cell penetration from the inner wall, growing outward was visible.

### Example 8

### Flow of Fluids Through the Walls of Nerve Tubes

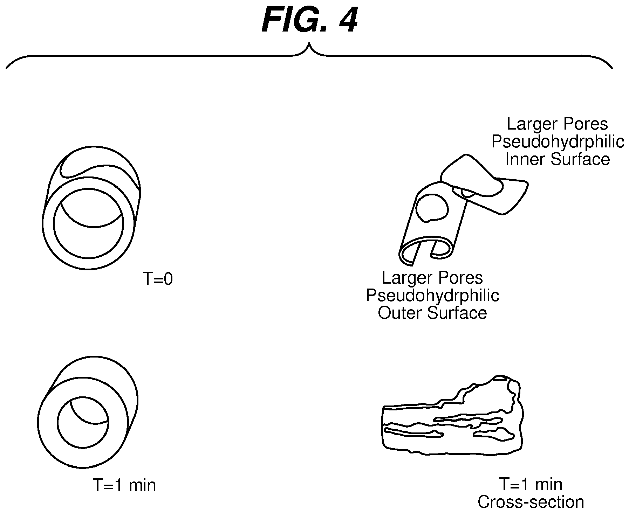

Nerve tubes of the present invention were tested for pore interconnectivity using a red (food) dye. Drops of fluids were added to the outer surface of the tube and the fluid flow was captured using images on the inner surface of the tube. As can be seen in FIG. 4, left bottom, the dye had made its way through the wall of the porous tube. This is indicative of the fact that, even though the surfaces have different porosities, the pores are interconnected, such that the media, nutrients and salts can pass inwardly across the pore structure from the outer surface, through the tube wall, through the inner surface, and to the inner cavity, where nerve regeneration occurs. Similarly, fluids can pass outwardly through the tube wall out through the outer surface. Meanwhile, the cells on the outer surface cannot penetrate the tube wall (shown in FIG. 3) and hence the scaffold acts as a barrier for inflammation and inflammatory cell migration into the "regeneration space" within the tube.

It can also be observed that the two surfaces, the inner surface and the outer surface, have different wettabilities (FIG. 4). The outer surface, owing to its limited pore size behaves in a hydrophobic manner, leading to bead formation from the dye. Meanwhile, the inner surface, owing to its well distributed large pore architecture, leads to immediate spreading of the dye through its pores, behaving in a hydrophilic manner.

### Example 9

### Nerve Repair Using Nerve Tube of the Present Invention

A trauma patient presents with an injury to the patient's femur and a resulting crush injury to the patient's underlying sciatic nerve. The patient has no sensory feeling or motor activity at the effector muscle below the site of the injury and standard testing procedures reveal a likely severed nerve. The patient is prepared for surgery in a conventional manner and is anesthetized in a conventional medically appropriate manner. The surgeon makes an appropriate incision and dissects tissue overlying the nerve until a sufficient length of the nerve is exposed. Upon observation, the surgeon determines that a section of the nerve approximately 3 cm in length is damaged. The crush injury leads to significant loss of the nerve, therefore a graft or scaffold would be needed for repair, unlike small linear cuts, which can be repaired by fine suturing. The surgeon removes the damaged length of nerve. The surgeon determines that coaptation of the resulting nerve stumps is not possible since it would be necessary to tension the nerve ends. The patient is not a candidate for an autograft because of size mismatch and unavailability of sufficient nerve to bridge the gap. An allograft is not available. The surgeon decides that the highest likelihood of nerve regeneration would occur by utilizing a nerve tube to connect the ends of the severed nerve. The surgeon is provided with a bioabsorbable nerve tube of the present invention manufactured in accordance with Example 1. The nerve tube has an inner diameter of 2.5 mm, and outer diameter of 5 mm, and a length of 5 cm. The nerve tube is installed in the following manner. The nerve tube is optionally trimmed to length as required. The proximal and the distal ends of the patient's nerve about the gap are inserted into the lumen of the tube and connected to the tube with a series of fine sutures. The muscle on the top is closed with sutures or staples and the outer skin and fascia are closed and sutured as well. After the nerve tube is affixed in place, the procedure is completed by approximating the incision in a medically acceptable, conventional manner using conventional surgical sutures, and the incision is appropriately bandaged.

Two months after the procedure, it is determined that the stumps of the sciatic nerve are connected and tests reveal that the patient has recovered at least partial motor function and almost complete sensory functions.

The novel porous nerve tubes and processes to manufacture such nerve tubes of the present invention have many advantages. The advantages include completely degradable matrix, minimal chances of immune rejection or reaction, no need for secondary surgery leading to donor site morbidity and functional loss. Also, the novel design also prevents migration of inflammatory cells into the regeneration cavity, while providing passage for nutrients through the scaffold into the tube, and egress of waste products and metabolites out through the wall of the porous tube. Also, if the cavities of the tubes are optionally filled with additional compositions such as gel-like materials including collagen or fibrin gels, the porous structure prevents leakage of the material out of the tube. The novel nerve tubes of the present invention are useful to repair nerve gaps of up to 30 mm, and it is believed that the nerve tubes may be useful to repair nerve gaps up to 50 mm or larger.

## Claims

1. A porous, bioabsorbable nerve tube structure comprising:
a bioabsorbable, polymeric tubular foam member comprising a lumen, an inner surface surrounding the lumen and an outer surface defining a wall, the wall having a thickness, wherein the substrate has a gradient of pore size from the interior surface to the exterior surface across the wall thickness of the tubular member such that tissue cells may infiltrate from the inner surface into the wall, and the outer surface is impermeable by cells, and the substrate is permeable by fluids through the wall of the foam member;
wherein the gradient of pore size comprises pores each having a diameter, the diameters ranging along the gradient from about 100 µm at the inner surface of the wall to about 1 µm at the outer surface of the wall.

2. A method of making a porous, bioabsorbable nerve tube structure, comprising the steps of:
providing a bioabsorbable polymer solution;
spin casting the polymer solution at an angular speed of 800 rpm to 3,000 rpm for 12 hours to 48 hours to form a tubular structure;
lyophilizing the tubular structure to form a nerve tube comprising a porous, bioabsorbable, polymeric tubular foam member comprising a lumen, an inner surface surrounding the lumen and an outer surface defining a wall, the wall having a thickness, wherein the substrate has a gradient of pore size from the interior surface to the exterior surface across the wall thickness of the tubular member such that tissue cells may infiltrate from the inner surface into the wall, and the outer surface is impermeable by cells, and the substrate is permeable by fluids through the wall.

3. The nerve tube of claim 1 or the method of claim 2, wherein the nerve tube comprises a synthetic polymer selected from the group consisting of polycaprolatone, polyglycolic acid, polyurethanes, polylactic acids and combinations thereof.

4. The nerve tube of claim 1 or the method of claim 2, wherein the nerve tube comprises a natural polymer selected from the group consisting of collagen, hyaluronic acid, fibrin, gelatin, elastin and other proteins.

5. The nerve tube or the method of claim 3, wherein the polymer additionally comprises an additive selected from the group consisting of growth factors, antibacterials, small molecules, natural compounds or polymers, cell based extracts, platelet extract, stem cell extract, neural cell extract, extracellular proteins, collagen, fibronectin, and laminin.

6. The method of claim 2, wherein the gradient of pore size comprises pores each having a diameter, the diameters ranging along the gradient from about 100 µm at the inner surface of the wall to about 1 µm at the outer surface of the wall.

7. The nerve tube of claim 1 or the method of claim 2, wherein the lumen is at least partially filled with a gel-like porous filler material selected from the group consisting of collagen, gelatin, fibrin, elastin, hyaluronic acid and other synthetic polymers.

8. The nerve tube of claim 1 or the method of claim 2, wherein the inner lumen comprises a structure selected from the group consisting of channels, fibers, hydrogels, and porous foams.

9. The nerve tube of claim 1 or the method of claim 2, wherein the nerve tube additionally comprises a medically useful substance.

10. The nerve tube or the method of claim 9, wherein the medically useful substance comprises a substance selected from the group consisting of growth factors, small molecules, antimicrobials, proteins and peptides.

11. The nerve tube or the method of claim 7, wherein the filler material additionally comprises a material from the group consisting of growth factors, controlled release formulations, proteins and peptides, small molecules and cell extracts.

12. The nerve tube or the method of claim 9, wherein the medically useful substance is selected from the group consisting of antimicrobials and therapeutic agents.

13. The nerve tube of claim 1 or the method of claim 2, additionally comprising a boundary layer in the wall between the inner surface and an outer surface.

14. The nerve tube of claim 1, wherein the outer surface is hydrophobic, and the inner surface is hydrophilic.

## Patentansprüche

1. Poröse, bioabsorbierbare Nervenrohrstruktur, umfassend:
ein bioabsorbierbares, polymeres, röhrenförmiges Schaumelement, umfassend ein Lumen, eine innere Oberfläche, die das Lumen umgibt und eine eine Wand definierende äußere Oberfläche, wobei die Wand eine Dicke aufweist, wobei das Substrat einen Porengrößengradienten von der inneren Oberfläche über die Wanddicke des röhrenförmigen Elements hinweg zur äußeren Oberfläche aufweist, so dass Gewebezellen von der inneren Oberfläche in die Wand eindringen können und die äußere Oberfläche für Zellen undurchlässig ist, und das Substrat durch die Wand des Schaumelements für Flüssigkeiten durchlässig ist;
wobei der Porengrößengradient Poren umfasst, die jeweils einen Durchmesser aufweisen, wobei die Durchmesser entlang des Gradienten von etwa 100 µm an der inneren Oberfläche der Wand bis zu etwa 1 µm an der äußeren Oberfläche der Wand reicht.

2. Verfahren zur Herstellung einer porösen, bioabsorbierbaren Nervenrohrstruktur, umfassend die Schritte:
Bereitstellen einer bioabsorbierbaren Polymerlösung;
Schleudergießen der Polymerlösung bei einer Winkelgeschwindigkeit von 800 U/min bis 3000 U/min für 12 Stunden bis 48 Stunden, so dass eine röhrenförmige Struktur gebildet wird;
Lyophilisieren der röhrenförmigen Struktur, um ein Nervenrohr zu bilden, das ein poröses, bioabsorbierbares, polymeres, röhrenförmiges Schaumelement umfasst, das ein Lumen, eine das Lumen umgebende innere Oberfläche und eine eine Wand definierende äußere Oberfläche umfasst, wobei die Wand eine Dicke aufweist, wobei das Substrat einen Porengrößengradient von der inneren Oberfläche über die Wanddicke des röhrenförmigen Elements zu der äußeren Oberfläche aufweist, so dass Gewebezellen von der inneren Oberfläche in die Wand eindringen können, und die äußere Oberfläche für Zellen undurchlässig ist, und das Substrat durch die Wand für Flüssigkeiten durchlässig ist.

3. Nervenrohr nach Anspruch 1 oder Verfahren nach Anspruch 2, wobei das Nervenrohr ein synthetisches Polymer umfasst, das aus der Gruppe ausgewählt ist, die aus Polycaprolaton, Polyglycolsäure, Polyurethanen, Polymilchsäuren und Kombinationen davon besteht.

4. Nervenrohr nach Anspruch 1 oder Verfahren nach Anspruch 2, wobei das Nervenrohr ein natürliches Polymer umfasst, das aus der Gruppe ausgewählt ist, die aus Kollagen, Hyaluronsäure, Fibrin, Gelatine, Elastin und anderen Proteinen besteht.

5. Nervenrohr oder Verfahren nach Anspruch 3, wobei das Polymer zusätzlich ein Additiv umfasst, das aus der Gruppe ausgewählt ist, die aus Wachstumsfaktoren, antibakteriellen Mitteln, kleinen Molekülen, natürlichen Verbindungen oder Polymeren, Extrakten auf Zellbasis, Plättchenextrakt, Stammzellextrakt, Neuralzellextrakt, extrazellulären Proteinen, Kollagen, Fibronectin und Laminin besteht.

6. Verfahren nach Anspruch 2, wobei der Porengrößengradient Poren umfasst, die jeweils einen Durchmesser aufweisen, wobei die Durchmesser entlang des Gradienten von etwa 100 µm an der inneren Oberfläche der Wand bis zu etwa 1 µm an der äußeren Oberfläche der Wand reicht.

7. Nervenrohr nach Anspruch 1 oder Verfahren nach Anspruch 2, wobei das Lumen mindestens teilweise mit einem gelartigen porösen Füllstoff gefüllt ist, der aus der Gruppe ausgewählt ist, die aus Kollagen, Gelatine, Fibrin, Elastin, Hyaluronsäure und anderen synthetischen Polymeren besteht.

8. Nervenrohrstruktur nach Anspruch 1 oder Verfahren nach Anspruch 2, wobei das innere Lumen eine Struktur umfasst, die aus der Gruppe ausgewählt ist, die aus Kanälen, Fasern, Hydrogelen und porösen Schäumen besteht.

9. Nervenrohr nach Anspruch 1 oder Verfahren nach Anspruch 2, wobei das Nervenrohr zusätzlich eine medizinisch nützliche Substanz umfasst.

10. Nervenrohr oder Verfahren nach Anspruch 9, wobei die medizinisch nützliche Substanz eine Substanz umfasst, die aus der Gruppe ausgewählt ist, die aus Wachstumsfaktoren, kleinen Molekülen, antimikrobiellen Wirkstoffen, Proteinen und Peptiden besteht.

11. Nervenrohr oder Verfahren nach Anspruch 7, wobei das Füllmaterial zusätzlich ein Material umfasst, das aus der Gruppe ausgewählt ist, die aus Wachstumsfaktoren, Formulierungen mit kontrollierter Freisetzung, Proteinen und Peptiden, kleinen Molekülen und Zellextrakten besteht.

12. Nervenrohr oder Verfahren nach Anspruch 9, wobei die medizinisch nützliche Substanz aus der Gruppe ausgewählt ist, die aus antimikrobiellen Wirkstoffen und therapeutischen Wirkstoffen besteht.

13. Nervenrohr nach Anspruch 1 oder Verfahren nach Anspruch 2, zusätzlich umfassend eine Grenzschicht in der Wand zwischen der inneren Oberfläche und einer äußeren Oberfläche.

14. Nervenrohr nach Anspruch 1, wobei die äußere Oberfläche hydrophob ist und die innere Oberfläche hydrophil ist.

## Revendications

1. Structure de tube nerveux biorésorbable poreuse comprenant :
un élément de mousse tubulaire polymère biorésorbable comprenant une lumière, une surface interne entourant la lumière et une surface externe définissant une paroi, la paroi ayant une épaisseur, dans laquelle le substrat a un gradient de taille de pore de la surface intérieure vers la surface extérieure à travers l'épaisseur de paroi de l'élément tubulaire de sorte que des cellules de tissu puissent infiltrer depuis la surface interne dans la paroi, et la surface externe soit imperméable aux cellules, et le substrat soit perméable aux fluides à travers la paroi de l'élément de mousse ;
dans laquelle le gradient de taille de pore comprend des pores ayant chacun un diamètre, les diamètres étant dans un gradient d'environ 100 µm à la surface interne de la paroi à environ 1 µm à la surface externe de la paroi.

2. Procédé de fabrication d'une structure de tube nerveux biorésorbable poreuse, comprenant les étapes de :
fourniture d'un solution de polymère biorésorbable ;
coulée par centrifugation de la solution de polymère à une vitesse angulaire de 800 tours/min à 3 000 tours/min pendant 12 heures à 48 heures pour former une structure tubulaire ;
lyophilisation de la structure tubulaire pour former un tube nerveux comprenant un élément tubulaire de mousse polymère biorésorbable poreux comprenant une lumière, une surface interne entourant la lumière et une surface externe définissant une paroi, la paroi ayant une épaisseur, dans lequel le substrat a un gradient de taille de pore de la surface intérieure vers la surface extérieure à travers l'épaisseur de paroi de l'élément tubulaire de sorte que des cellules de tissu puissent infiltrer depuis la surface interne dans la paroi, et la surface externe soit imperméable aux cellules, et le substrat soit perméable aux fluides à travers la paroi.

3. Tube nerveux de la revendication 1 ou procédé de la revendication 2, le tube nerveux comprenant un polymère synthétique choisi dans le groupe constitué de la polycaprolatone, de l'acide polyglycolique, de polyuréthanes, d'acides polylactiques et de combinaisons de ceux-ci.

4. Tube nerveux de la revendication 1 ou procédé de la revendication 2, le tube nerveux comprenant un polymère naturel choisi dans le groupe constitué du collagène, de l'acide hyaluronique, de la fibrine, de la gélatine, de l'élastine et d'autres protéines.

5. Tube nerveux ou procédé de la revendication 3, le polymère comprenant en outre un additif choisi dans le groupe constitué de facteurs de croissance, agents antibactériens, petites molécules, composés ou polymères naturels, extraits à base de cellules, extrait de plaquettes, extrait de cellules souches, extrait de cellules neuronales, protéines extracellulaires, collagène, fibronectine et laminine.

6. Procédé de la revendication 2, dans lequel le gradient de taille de pore comprend des pores ayant chacun un diamètre, les diamètres étant dans un gradient d'environ 100 µm à la surface interne de la paroi à environ 1 µm à la surface externe de la paroi.

7. Tube nerveux de la revendication 1 ou procédé de la revendication 2, la lumière étant au moins partiellement remplie d'un matériau de remplissage poreux de type gel choisi dans le groupe constitué du collagène, de la gélatine, de la fibrine, de l'élastine, de l'acide hyaluronique et d'autres polymères synthétiques.

8. Tube nerveux de la revendication 1 ou procédé de la revendication 2, la lumière interne comprenant une structure choisie dans le groupe constitué de canaux, fibres, hydrogels et mousses poreuses.

9. Tube nerveux de la revendication 1 ou procédé de la revendication 2, le tube nerveux comprenant en outre une substance médicalement utile.

10. Tube nerveux ou procédé de la revendication 9, la substance médicalement utile comprenant une substance choisie dans le groupe constitué de facteurs de croissance, petites molécules, agents antimicrobiens, protéines et peptides.

11. Tube nerveux ou procédé de la revendication 7, le matériau de remplissage comprenant en outre un matériau dans le groupe constitué de facteurs de croissance, formulations à libération contrôlée, protéines et peptides, petites molécules et extraits de cellules.

12. Tube nerveux ou procédé de la revendication 9, la substance médicalement utile étant choisie dans le groupe constitué d'agents antimicrobiens et d'agents thérapeutiques.

13. Tube nerveux de la revendication 1 ou procédé de la revendication 2, comprenant en outre une couche limite dans la paroi entre la surface interne et une surface externe.

14. Tube nerveux de la revendication 1, dans lequel la surface externe est hydrophobe, et la surface interne est hydrophile.
